# EUROPEAN PATENT APPLICATION

(11) **EP 2 128 133 A1**
(43) Date of publication of application: **02.12.2009**
(21) Application number: 08156895.8
(22) Date of filing: 26.05.2008
(51) Int. Cl.: C07D 205/08

(54) **Ezetimibe process and composition**

(71) Applicant: Lek Pharmaceuticals D.D., 1526 Ljubljana (SI)
(72) Inventor: Selic, Lovro, 3000, Celje (SI)
(74) Representative: Kunic, Barbara

(57) **Abstract**

The present invention describes a process for producing ezetimibe (EZT) from a protected compound, including a step of deprotecting the 4-(p-hydroxyphenyl) protection group by catalytic hydrogenation, wherein the catalyst is used in an amount of 0.7 wt.-% or lower, relative to the weight of the compound used for the deprotection reaction. After carrying out a step of deprotection reaction, the process preferably comprises: (a) the reaction product is dissolved or extracted in ethyl acetate, and (b) the ethyl acetate solution is washed with an aqueous salt solution. The invention further describes a process for obtaining pure EZT, wherein raw EZT is dissolved in a solvent at a concentration of lower than 0.1 g/ml, and a crystallization step is carried out from this solution. These measures, respectively alone and particularly in combination contribute to attain ezetimibe (EZT) free of critical impurities described herein, and thus to use exceptionally pure ezetimibe (EZT) to be formulated into a pharmaceutical composition together with a pharmaceutically acceptable carrier or excipient.

## Description

### Field of the invention

The present invention relates a process for producing or preparing ezetimibe (in the following sometimes abbreviated as EZT) in pure form, and to exceptionally pure ezetimibe (EZT) as well as a pharmaceutical composition containing the same together with a pharmaceutically acceptable carrier and/or excipient.

### Background of the invention

Ezetimibe (EZT), chemically defined as (3R,4S)-1-(p-fluorophenyl)-3-((3S)-3-(p-fluorophenyl)-3-hydroxypropyl)-4-(p-hydroxyphenyl)-2-azetidinone, is known as a useful drug, in particular as anticholesteremic agent, as antihyperlipidemic agent [notably an intestinal cholesterol absorption inhibitor], as antilipemic agent and as antimetabolite.

In the synthesis of ezetimibe, deprotection of the hydroxy-group of the 4-(p-hydroxyphenyl) structural moiety of EZT is normally the last step in the synthetic pathway. Deprotection by removal of OH-protecting group is a well-known organic reaction and is typically carried out by catalytic hydrogenation using a suitable catalyst and a source of hydrogen. Synthetic pathways and deprotection steps are, for example, disclosed in EP 720 599 A, WO 2004/99132 A2 and US 2007/0049748 A1.

The object of the present invention was to provide an improved process for producing ezetimibe (EZT), and to make pure and preferably exceptionally pure ezetimibe (EZT) feasible, and to enable use of such exceptionally pure EZT as a starting compound for obtaining more safe pharmaceutical compositions comprising EZT.

### Summary of the Invention

In a first aspect, the present invention provides a process for producing ezetimibe of Formula II (EZT) from a compound of Formula I, including a step of deprotecting the OR protection group by catalytic hydrogenation, wherein a metal catalyst is used in an amount of 0.7 wt.-% or lower, relative to the weight of the compound of Formula I used for the deprotection reaction:

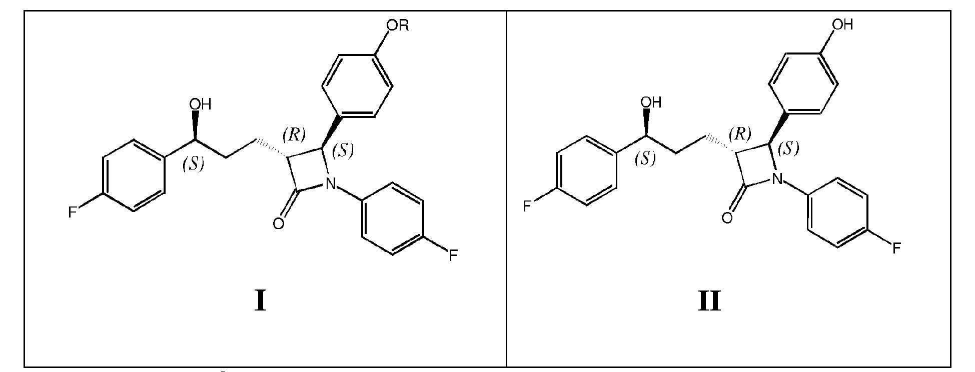

wherein R denotes OH-protection group.

According to another aspect, the present invention provides a process for obtaining (3R, 4S)-1-(p-fluorophenyl)-3-((3S)-3-(p-fluorophenyl)-3-hydroxypropyl)-4-(p-hydroxyphenyl)-2-azetidinone (ezetimibe [EZT]) from the 4-(p-hydroxyphenyl)-protected precursor compound, wherein after carrying out a step of deprotection reaction of the 4-(p-hydroxyphenyl) hydroxy-group, the process comprises the following steps:
(a) the reaction product is dissolved or extracted in ethyl acetate, and
(b) the ethyl acetate solution obtained in step (a) is washed with an aqueous salt solution.

According to yet an another aspect, the present invention provides a process for obtaining pure (3R,4S)-1-(p-fluorophenyl)-3-((3S)-3-(p-fluorophenyl)-3-hydroxypropyl)-4-(p-hydroxyphenyl)-2-azetidinone (ezetimibe [EZT]), wherein raw ezetimibe (EZT) is dissolved in a solvent at a concentration of lower than 0.1 g/ml solution, and a crystallization step is carried out from this solution.

Observing the conditions of the aforementioned aspects of the processes according to the present invention in combination makes it feasible to obtain hitherto unattainable, exceptionally pure ezetimibe (EZT) essentially free, and even entirely free, of the compound of Formula III:

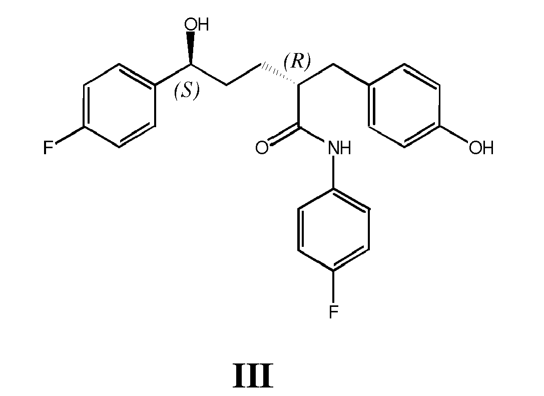

Thus, the present invention makes it feasible to use exceptionally pure ezetimibe essentially free, preferably entirely free of the compound of Formula III as a starting compound to be formulated into a pharmaceutical composition together with at least one pharmaceutically acceptable carrier and/or excipient. Most advantageously, the EZT active ingredient is one which had been subjected to all procedural measures of deprotection reaction, extraction into ethyl acetate and washing with aqueous salt solution, as well as crystallization as defined in the above-described first to third aspects of the process according to the present invention.

The term "essentially free" used herein means less than 0.2 wt.-%, preferably less than 0.1 wt.-%, preferably less than 0.05 wt.-% of the compound of Formula III relative to the total weight of the yielded EZT product. The term "entirely free" means below the detection limit using methods of ¹H- or ¹³C-NMR.

### Brief description of the Drawings

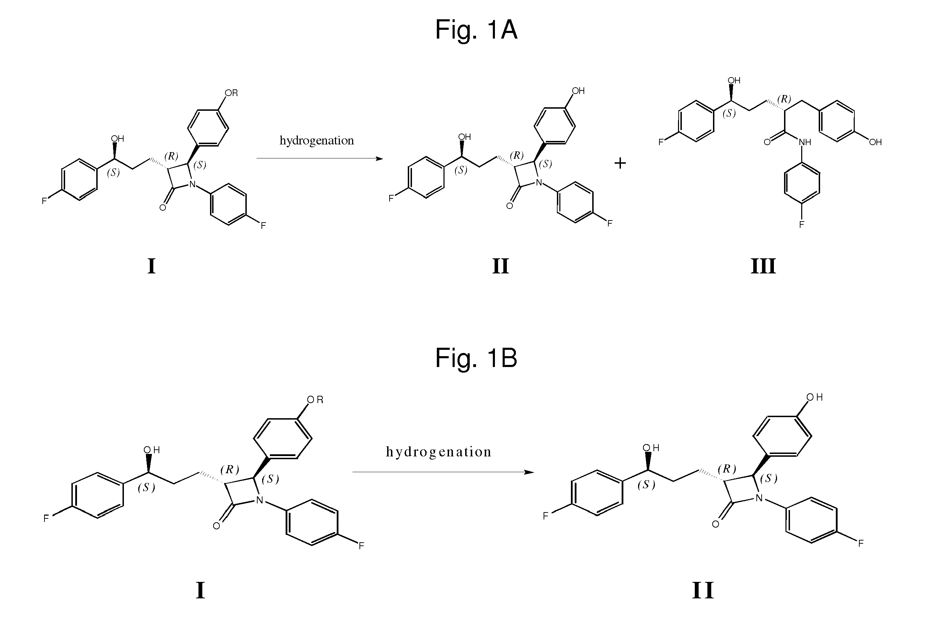

Fig. 1 A schematically shows a reaction scheme for catalytic hydrogenation of the 4-(p-hydroxyphenyl)-protected precursor compound of EZT with Formula I without proper control of the reaction conditions, whereby substantial amounts of impurity EZT-FAM of Formula III is inevitably generated besides desired product EZT of formula II, whereas
Fig. 1B schematically shows a corresponding reaction scheme involving catalytic hydrogenation of the 4-(p-hydroxyphenyl)-protected precursor compound of EZT with Formula I, but with proper control of the reaction conditions, whereby impurity EZT-FAM of Formula III is avoided or minimized and yet the yield of the desired product EZT of formula II is substantially increased.

### Description of Preferred Embodiments

In the present invention, it was surprisingly found that a yet unknown or hidden, but critical impurity is generated in the deprotection reaction using catalytic hydrogenation. The critical impurity was identified as a product of secondary hydrogenation to eventually yield EZT-FAM (compound of Formula III), as shown in reaction scheme in Fig. 1A. Owing to this basic recognition, it was surprisingly found according to the present invention that reducing the amount of metal catalyst used in the catalytic hydrogenation reaction yields the desired EZT with minimum amount of EZT-FAM possible, as illustrated schematically in the reaction scheme of Fig. 1B. Accordingly, in the first aspect the process of the present invention is significantly improved by using in the deprotection reaction an amount of metal catalyst of lower than previously described in the above-referenced prior art, specifically 0.7 wt.-% or lower, preferably 0.6 wt.-% or lower, more preferably 0.3 wt.-% or lower and particularly preferably 0.1 wt.-% or lower, respectively relative to the weight amount of the protected EZT compound. A lower limit of metal catalyst may depend on the minimum amount still effective for catalysing the dehydrogenation reaction - which may also be affected by further reaction conditions. A suitable lower limit is, for example, 0.001 wt.-% relative to the weight amount of the protected EZT compound. A particularly preferred range of the catalyst is 0.01 to 0.1 wt.%, relative to the weight amount of the protected EZT compound.

It was furthermore surprisingly found that extraction of EZT product with ethyl acetate and subsequently washing with aqueous salt solution, and crystallization of purified raw EZT from a solution containing low concentrations of EZT of lower than 0.1 g/ml solution, respectively alone and preferably in combination further substantially reduces EZT-FAM. In combination with the control of metal catalyst in the preceding deprotecting reaction described above, it is even feasible to achieve entire freeness of this critical impurity, i.e. a level substantially undetectable by methods of ¹H- and ¹³C-NMR and possibly mass spectrometry. In order to be not mistaken with other possible impurities such as EZT-AOL (see the compound of Formula IV) that has identical mass, ¹³C-NMR is the preferred reference method to confirm absence of the critical impurity EZT-FAM, and possibly other impurities or side products. Most advantageously, it is feasible according to the present invention to provide exceptionally pure ezetimibe (EZT) being entirely free of EZT-FAM (compound of Formula III), i.e. having zero or at most undetectable levels of EZT-FAM. Further advantageously, the processes according to the invention enable to obtain EZT being substantially free and even completely free also of other impurities.

Thus, each of the following three technical concepts respectively contribute significantly to yielding EZT in hitherto unattainable, exceptionally pure form:
1. particular reaction condition during the deprotection reaction as the final, in terms of impurities yet most crucial synthesis step in the EZT synthesis pathway, to minimize the amount of EZT-FAM and other side products;
2. combination of a particular purification steps involving extraction and washing, which removes most of the still remaining EZT-FAM; and
3. particular crystallization procedure, which completely removes EZT-FAM and all other impurities, including diastereomers of ezetimibe.

It is important to note that the afore-mentioned technical concepts 2 and 3 alone or in combination would yet be insufficient to make the yield of exceptionally pure ezetimibe according to the present invention feasible because these may only relatively reduce the ratio of EZT-FAM and other impurities relative to EZT, but not absolutely to a level close to or even reaching zero (i.e. at a maximum of being undetectable by e.g. ¹³C-NMR). For this to achieve, it is important that the amount of EZT-FAM and other side products are surely minimized by using substantially reduced amount of catalyst during the catalytic hydrogenation reaction representing the most critical last step in the synthesis pathway of EZT. Even in case that the reaction conditions in technical concept 1 is not properly observed, concept 2 and/or concept 3 still lead to improvement of in EZT production processes.

Even though the loading amount of catalyst is reduced, preferably substantially reduced according to the first aspect of the present invention in the catalytic hydrogenation of the deprotection step, the yield of the deprotection procedure surprisingly far exceeds previously reported yields of EZT, probably thanks to the minimization and finally complete removal of EZT-FAM and possible other side products. Thus, not only by the need of lower amounts of expensive catalysts, but also the substantially improved yield of more than 50 %, preferably more than 70 % and even reaching at least 75 % molar percentage of the OR-protected starting compound, the processes according to the present invention displays a particular advantageous economical process for obtaining EZT.
The term "OR protection group" used herein means a hydroxyl protection group, typically one which is susceptible to deprotection by catalytic hydrogenation.

Particularly suitable OR protection groups include, but are not limited to benzyl (Bn), benzyl carbonate (Cbz), benzyloxymethylether (BOM), paramethoxybenzyl ether (PMB) and the like.

The deprotection reaction according to the present invention most preferably involves deprotection of a benzyl protecting group present at the 4-(p-hydroxyphenyl) structural moiety of EZT (in the following sometimes abbreviated as BnEZT).

As the source of hydrogen for the catalytic hydrogenation, H₂ gas; ammonium formate plus formic acid; cyclohexene; cyclohexadiene, diimide; and the like may be used, but is not limited thereto.

The catalyst for the deprotection reaction based on catalytic hydrogenation can include, but is not limited to metals such as Pt, Pd and Rh or transition metals like Mo, W, Cr, Fe, Co and Ni, respectively alone or in combination. For enhancing activity and/or stability of the catalyst, it can be used in combination with a suitable support material such as, for example activated carbon, alumina, silica, without being limited thereto. Preferred loading amount of metal catalyst on solid support is 5 wt.-% in order to further control activity. Preferably, palladium and more preferably palladium on carbon is used as the catalyst, with a preferred loading of palladium on carbon being 5 %.

In the extraction and washing procedure of the particular aspect process according to present invention, it was surprisingly found that a critical impurity in form of EZT-FAM and others can be substantially reduced by up to about 10 % by weight. The choice of ethyl acetate as the extraction solvent effectively and selectively dissolves EZT against EZT-FAM, and in combination with subsequent washing by aqueous salt solution further minimizes other impurities and side products. A suitable aqueous salt solution is aqueous NaCl, in particular brine with a content of at least 5 %, more preferably at least 10 % by weight NaCl. A particularly suitable washing solution is saturated brine.

Provided that the preceding steps of critical reaction conditions during deprotection by catalytic hydrogenation and subsequent extraction and washing procedure are carried out, the subsequent crystallization can be performed in such a way that remainders of EZT-FAM and all other impurities can be completely removed, including diastereomers of ezetimibe. Performing crystallization at low concentrations, preferably at a concentration lower than 0.1 g/ml and more preferably lower than 0.05 g/ml, in particular in a concentration range of from about 0.005 g/ml to 0.025 g/ml relative to the entire volume of the crystallization solution is the last critical step to be performed in combination to obtain yet unattainable, exceptionally pure ezetimibe. Suitable solvents, from which EZT can be crystallized, include toluene, xylene, cyclohexane, hexane, without being limited thereto. A solvent in which ezetimibe is poorly soluble, such as toluene as the most preferred one, is preferred in the crystallization step thanks to high yields achievable by the crystallization. For further improved yield, the solvent should be adjusted to cold temperature, suitably to below 10 °C, preferably to lie in a range of about 0 to about 4 °C. The described crystallization step can be repeated, preferably 2 or 3 times.

The exceptionally pure ezetimibe thus provided by the present invention can be advantageously formulated into a pharmaceutical composition together with at least one pharmaceutically acceptable carrier and/or excipient. The pharmaceutical composition can be used for the prevention and/or the treatment of any one of diseases selected form cholesteremic, hyperlipidemic and lipemic conditions, cancers and antibiotic conditions. A preferred use of application is hypercholesterolemia. A suitable dosage of ezetimibe may lie in a range of 0.1 mg to 100 mg, more suitably of 1 mg to 20 mg of the whole pharmaceutical composition. The pharmaceutically acceptable carrier and/or excipient can be chosen from suitable carriers and excipients known to the person skilled in the art. For example, suitable pharmaceutically acceptable carriers and/or excipients may include, but are not limited to matrix forming agents, diluents, antioxidants, bufferants, antifoaming agents, detackifiers, preservatives, chelating agents, viscomodulators, tonicifiers, flavorants, colorants, odorants, opacifiers, stabilizing agents, solubilizers, binders, fillers, plasticizing agents, lubricants, and mixtures thereof.

The carriers and excipients may also be chosen depending on the desired administration and dosage form. Suitable administration forms include orally, parenterally, through injection, transdermally, nasally, rectally, through inhalation, without being limited thereto. Suitable dosage forms include tablets, capsules, granulations, powders, pellets, suspensions, liquids, suppositories, sustained release dosage forms, without being limited thereto. In the pharmaceutical preparation according to the present invention, ezetimibe may also be combined with one or more other active ingredients.
The present invention is described in further detail below by referring to examples, which however are provided only for illustrative purposes and the invention is in no way limited thereto.

### EXAMPLE 1

To 20g [10 mmol, 1 EQ] BnEZT, suspended in 400 ml of methanol, 25 g of ammonium formate [9.9 EQ] was added, and the suspension was deaerated with argon or nitrogen. Reaction mixture was heated to 35°C, then 0.24 g Pd(C) (5 % Fluka), corresponding to 0.06 wt.-% relative to the amount of BnEZT used, and 2 ml formic acid [1.3 EQ] was added. Reaction was maintained at 35°C for 6-7h, and thereafter filtrated through celite. Filtrate was evaporated. The product was extracted by dissolution in 400 ml ethyl acetate (EtOAc), and the EtOAc phase was then washed with 400 ml brine (10 %). Organic phase was concentrated. Solid residue was 2-3x crystallized from toluene (50 ml/g): Crystals/precipitate were collected by filtration. Subsequently they were dissolved in 2-propanol (7.5 ml/g), then slowly precipitated by addition of water (30 ml/g). Precipitate is collected and dried in vacuum, to give 12.4g (75 %) of exceptionally pure ezetimibe.
C₂₄H₂₁ F₂NO₃
M.W. 409,43
¹**H NMR** (300MHz, DMSO-d₆): δ 1.73-1.88 (m, 4H), 3.08 (m, 1 H), 4.50 (d, 1 H, 3.4), 4.79 (d, 1 H, 2.1), 5.29 (d, 1 H, 4.1), 6.77 (d, 2H, 8.6), 7.07-7.33 (m, 10H), 9.54 (s, 1 H)
**¹³C NMR** (75MHz, DMSO-d₆): 824.6, 36.4, 59.5, 59.6, 71.1, 114.7 (21 Hz), 115.8, 115.9 (23Hz), 118.3 (8Hz), 127.6 (8Hz), 127.6, 127.9, 130.0 (2Hz), 142.2 (2Hz), 157.9 (227Hz), 157.5, 161.3 (228Hz), 167.4.

### EXAMPLE 2

To 5 g [1 EQ] BnEZT, suspended in 85 ml of methanol, 6.3 g of ammonium formate [10 EQ] was added, and the suspension was deaerated with argon or nitrogen. After that, 3.6 g Pd(C) (5 % Fluka) and 0.5 ml of formic acid [1.3 EQ] were added. Reaction was maintained at room temperature for 30' and filtrated through celite. Filtrate was evaporated. The product was extracted by dissolution in EtOAc, and the EtOAc phase was then washed with brine (10 %). Organic phase was concentrated. Upon crystallization from toluene, both EZT and EZT-FAM could be isolated.

### Isolating pure crystallized EZT-FAM yields:

C₂₄H₂₃F₂NO₃
M.W. 411,16
**¹H NMR** (300MHz, DMSO-d₆): δ 1.49-1.82 (m, 4H), 2.48-2.53 (m, 2H), 2.76 (m, 1 H), 4.47 (m, 1 H), 5.20 (d, 4.5Hz, 1 H), 6.62 (d, 8.4Hz, 2H), 6.95 (d, 8.4Hz, 2H), 7.08 (m, 4H), 7.26 (m, 2H), 7.54 (m, 2H), 9.13 (bs, 1 H), 9.79 (bs, 1 H).
**¹³C NMR** (75MHz, DMSO-d₆): 828.5, 37.2, 37.7, 48.8, 71.4, 114.6 (21 HZ), 114.9, 115.2 (22.6 Hz), 121.0 (8Hz), 127.6 (8Hz), 129.7, 135.5 (2Hz), 142.3 (2Hz), 155.5, 157.8 (234 Hz), 161.1 (236 Hz), 173.4.

### EXAMPLE 3

To 5 g [1EQ] BnEZT, suspended in 85 ml of methanol, 6.3 g of ammonium formate [10 EQ] was added, and the suspension was deaerated with argon or nitrogen. After that, 0.6 g Pd(C) (5 % Fluka), corresponding to 0.12 wt.-% relative to BnEZT, and 0.5 ml of formic acid [1.3 EQ] were added. Reaction was maintained at room temperature for 3 h and filtrated through celite. Filtrate consisted of predominantly of ezetimibe and FAM as impurity.
Filtrate was evaporated. The product was extracted by dissolution in EtOAc, and the EtOAc phase was then washed with brine (10 %). Organic phase was concentrated. It consisted of almost pure ezetimibe and FAM as a substantially further reduced impurity.

## Claims

1. Process for producing ezetimibe of Formula II (EZT) from a compound of Formula I, including a step of deprotecting the OR-protecting group by catalytic hydrogenation, wherein the catalyst is a metal catalyst used in an amount of 0.7 wt.-% or lower, relative to the weight of the compound of Formula I used for the deprotection reaction: 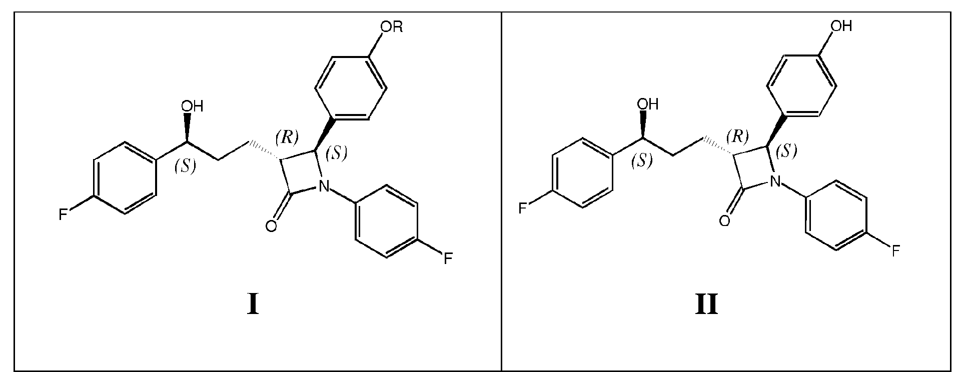 wherein R denotes OH-protection group.

2. The process according to claim 1, wherein R denotes benzyl as the OH-protecting group.

3. The process according to claim 1 or 2, wherein the metal catalyst is palladium.

4. The process according to any one of the preceding claims, wherein the metal catalyst is loaded on support, preferably at a loading amount of at most 5 wt.-% relative tot the total amount of the catalyst support.

5. The process according to any one of the preceding claims, wherein the metal catalyst is used at an amount of 0.6 wt.-% or lower, preferably 0.3 wt.-% or lower and particularly preferably 0.1 wt.-% or lower, respectively relative to the weight amount of the protected EZT compound.

6. Process for obtaining (3R,4S)-1-(p-fluorophenyl)-3-((3S)-3-(p-fluorophenyl)-3-hydroxypropyl)-4-(p-hydroxyphenyl)-2-azetidinone (ezetimibe, [EZT]) from the 4-(p-hydroxyphenyl)-protected precursor compound, wherein after carrying out a step of deprotection reaction of the 4-(p-hydroxyphenyl)hydroxy-group, the process comprises the following steps:
(a) the reaction product is dissolved or extracted in ethyl acetate, and
(b) the ethyl acetate solution obtained in step (a) is washed with an aqueous salt solution.

7. The process according to claim 6, wherein the aqueous salt solution is brine.

8. The process according to claim 6 or 7, wherein the step of deprotection reaction of the 4-(p-hydroxyphenyl)hydroxy-group has been carried out by a process according to any one of claims 1 to 5.

9. Process for obtaining pure (3R,4S)-1-(p-fluorophenyl)-3-((3S)-3-(p-fluorophenyl)-3-hydroxypropyl)-4-(p-hydroxyphenyl)-2-azetidinone (ezetimibe, [EZT]), wherein raw ezetimibe (EZT) is dissolved in a solvent at a concentration of lower than 0.1 g/ml solution, and a crystallization step is carried out from this solution.

10. The process according to claim 9, wherein the solvent for dissolving the EZT product is toluene.

11. The process according to any one of the preceding claims, wherein the finally obtained EZT product is essentially free of the compound of formula III by containing less than 0.2 wt.-%, preferably less than 0.1 wt.-%, more preferably less than 0.05 wt.-% of the compound of Formula III relative to the total weight of the yielded EZT product:

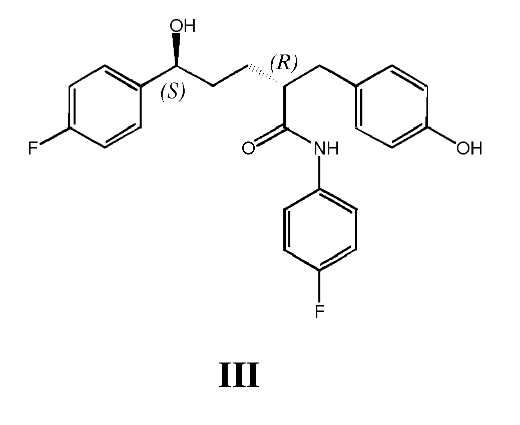

12. (3R,4S)-1-(p-fluorophenyl)-3-((3S)-3-(p-fluorophenyl)-3-hydroxypropyl)-4-(p-hydroxyphenyl)-2-azetidinone (ezetimibe [EZT]), being essentially free of the compound of formula III by containing less than 0.2 wt.-%, preferably less than 0.1 wt.-%, more preferably less than 0.05 wt.-% of the compound of Formula III relative to the total weight of the yielded EZT product:

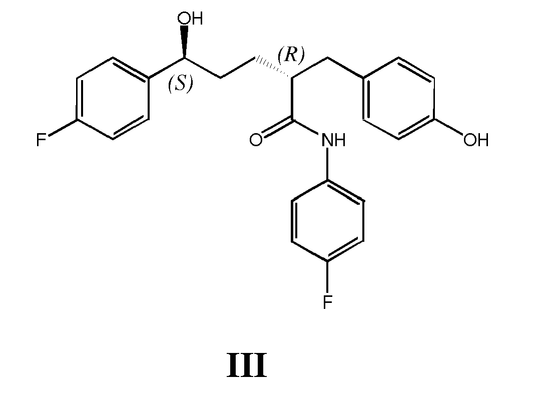

13. (3R,4S)-1-(p-fluorophenyl)-3-((3S)-3-(p-fluorophenyl)-3-hydroxypropyl)-4-(p-hydroxyphenyl)-2-azetidinone (ezetimibe [EZT]), being entirely free of the compound of formula III in terms of being undetectable by ¹H- and ¹³C-NMR and HPLC:

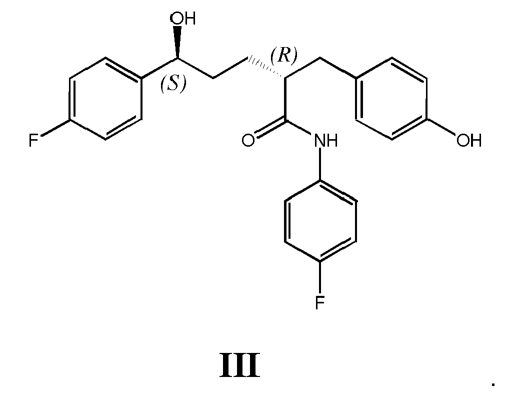

14. A pharmaceutical composition comprising (3R,4S)-1-(p-fluorophenyl)-3-((3S)-3-(p-fluorophenyl)-3-hydroxypropyl)-4-(p-hydroxyphenyl)-2-azetidinone (ezetimibe [EZT]) and a pharmaceutically acceptable carrier and/or excipient, wherein the ezetimibe formulated into the pharmaceutical composition was essentially free of the compound of formula III by containing less than 0.2 wt.-% of the compound of Formula III relative to the total weight of the yielded EZT product, wherein preferably the ezetimibe was entirely free of the compound of formula III in terms of being undetectable by ¹H- and ¹³C-NMR:

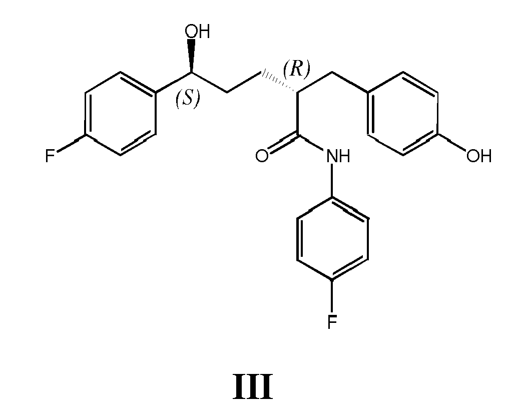

15. The pharmaceutical composition according to claim 14, wherein the ezetimibe formulated into the pharmaceutical composition was obtained by a process as defined in any one of claims 1 to 11.
